# EUROPÄISCHE PATENTSCHRIFT

(11) **EP 0 648 772 B1**
(45) Veröffentlichungstag und Bekanntmachung des Hinweises auf die Patenterteilung: **04.09.2002**
(21) Anmeldenummer: 94115645.7
(22) Anmeldetag: 05.10.1994
(51) Int. Cl.: C07D 513/04, C07D 231/04, C07D 237/04, C07D 207/22, C07D 211/72, C07C 331/28, C07C 311/08, C07C 255/60, A01N 43/90

(54) **4-Cyanophenyliminoheterocyclen**
4-Cyanophenyliminoheterocycles
4-Cyanophényliminohétérocycles

(30) Priorität: 18.10.1993 DE 4335438
(43) Veröffentlichungstag der Anmeldung: 19.04.1995
(60) Teilanmeldung: 01122556.2
(73) Patentinhaber: BAYER AG, 51368 Leverkusen (DE)
(72) Erfinder: Schallner, Otto Dr., D-40789 Monheim (DE); Andree, Roland Dr., D-40764 Langenfeld (DE); Drewes, Mark Wilhelm Dr., D-40764 Langenfeld (DE); Dollinger, Markus Dr., D-51381 Leverkusen (DE); Santel, Hans-Joachim Dr., D-51371 Leverkusen (DE)

(56) Entgegenhaltungen:
- EP-A- 0 238 711
- EP-A- 0 273 417
- EP-A- 0 304 920
- EP-A- 0 312 064
- EP-A- 0 410 265
- EP-A- 0 457 714
- EP-A- 0 494 819
- EP-A- 0 509 398
- WO-A-92/21684
- WO-A-93/05037
- DE-A- 2 153 602
- DE-A- 4 131 579
- GB-A- 1 194 835

## Beschreibung

Die Erfindung betrifft neue 4-Cyanophenyliminoheterocyclen, Verfahren zu ihrer Herstellung und ihre Verwendung als Herbizide sowie neue Zwischenprodukte für die Synthese.

Es ist bereits bekannt, daß bestimmte Halogenaryliminoheterocyclen herbizide Eigenschaften aufweisen (vgl. EP-A 238711, EP-A 273417, EP-A 312064, EP-A 410265, EP-A 457714, WO-A 92/21684).

Die herbizide Wirksamkeit bzw. die Verträglichkeit dieser bekannten Verbindungen gegenüber Kulturpflanzen ist jedoch nicht immer ganz zufriedenstellend.

Es wurden nun neue 4-Cyanophenyliminoheterocyclen der allgemeinen Formel (I) gefunden, 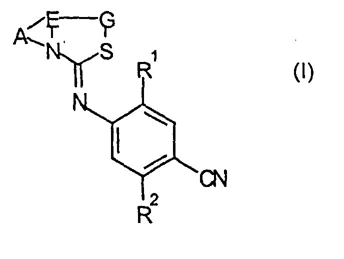 in welcher
- R¹: für Wasserstoff, Fluor, Chlor oder Brom, steht,
- R²: -N(R⁴)-SO₂R⁵ steht, wobei
- R⁴: für Wasserstoff oder für Alkyl mit 1 bis 8 Kohlenstoffatomen steht und
- R⁵: für gegebenenfalls durch Fluor, Chlor, Brom, Cyano, Carboxy, C₁-C₄-Alkoxy, C₁-C₄-Alkoxy-C₁-C₄-alkoxy, C₁-C₄-Alkylthio, C₁-C₄-Alkyl-carbonyl oder C₁-C₄-Alkoxy-carbonyl substituiertes Alkyl mit 1 bis 8 Kohlenstoffatomen steht,
- R⁵: weiterhin für jeweils gegebenenfalls durch Fluor, Chlor oder Brom substituiertes Alkenyl oder Alkinyl mit jeweils 2 bis 8 Kohlenstoffatomen steht,
- R⁵: weiterhin für jeweils gegebenenfalls durch Fluor, Chlor, Brom, Cyano, Carboxy, C₁-C₄-Alkyl oder C₁-C₄-Alkoxy-carbonyl substituiertes Cycloalkyl oder Cycloalkylalkyl mit jeweils 3 bis 8 Kohlenstoffatomen im Cycloalkylteil und gegebenenfalls 1 bis 4 Kohlenstoffatomen im Alkylteil oder für jeweils gegebenenfalls durch Fluor, Chlor, Brom, Cyano, Nitro, Carboxy, Carbamoyl, durch C₁-C₄-Alkyl, C₁-C₄-Alkoxy, C₁-C₄-Alkylthio, C₁-C₄-Alkylsulfinyl oder C₁-C₄-Alkylsulfonyl (welche jeweils gegebenenfalls durch Fluor und/oder Chlor substituiert sind), durch Dimethylaminosulfonyl oder Diethylaminosulfonyl, durch C₁-C₄-Alkoxy-carbonyl (welches gegebenenfalls durch Fluor, Chlor, Brom, Methoxy oder Ethoxy substituiert ist), durch Phenyl, Phenylmethyl, Phenoxy, Phenylthio, Phenylsulfinyl oder Phenylsulfonyl (welche jeweils gegebenenfalls durch Fluor, Chlor, Brom, Cyano, Methyl, Methoxy, Trifluormethyl, Difluormethoxy und/oder Trifluormethoxy substituiert sind) substituiertes Aryl oder Arylalkyl mit 6 oder 10 Kohlenstoffatomen im Arylteil und gegebenenfalls 1 bis 4 Kohlenstoffatomen im Alkylteil steht,
- A: für Alkandiyl oder Alkendiyl mit jeweils 4 Kohlenstoffatomen steht,
- E: für Stickstoff steht und
- G: für Kohlenstoff steht, welcher über eine exo-Doppelbindung mit Sauerstoff verbunden ist.

Man erhält die neuen 4-Cyanophenyliminoheterocyclen der allgemeinen Formel (I), wenn man
a) substituierte Thiocarbonylaminoverbindungen der allgemeinen Formel (III) 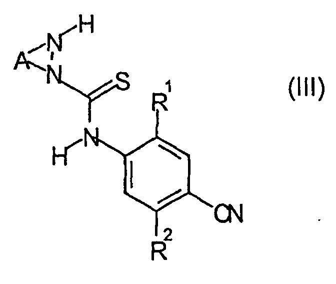 in welcher
   - A, R¹ und R²: die oben angegebenen Bedeutungen haben,
   mit Phosgen, gegebenenfalls in Gegenwart eines Verdünnungsmittels umsetzt,
   oder wenn man
b) 4-Cyanophenyliminoheterocyclen der allgemeinen Formel (Ia) 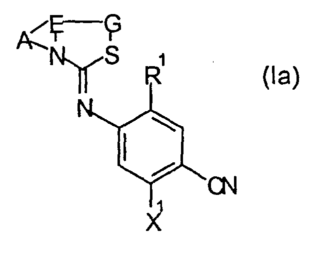
in welcher
- R¹, A, E und G: die oben angegebenen Bedeutungen haben und
- X¹: für Halogen steht,
mit nucleophilen Verbindungen der allgemeinen Formel (V)

H-N(R⁴)-SO₂R⁵ (V)

in welcher
- R⁴ und R⁵: die oben angegebenen Bedeutungen haben,
- oder mit Alkalimetallsalzen dieser Verbindungen -
gegebenenfalls in Gegenwart eines Säureakzeptors und gegebenenfalls in Gegenwart eines Verdünnungsmittels umsetzt.

Die neuen Verbindungen der Formel (I) können auch durch Umsetzung von Iminoheterocyclen der allgemeinen Formel (VI) mit 4-Halogen-benzonitrilen der allgemeinen Formel (VII) gemäß folgendem Formelschema erhalten werden (R¹, R², A, E und G wie oben definiert, X²: Halogen):

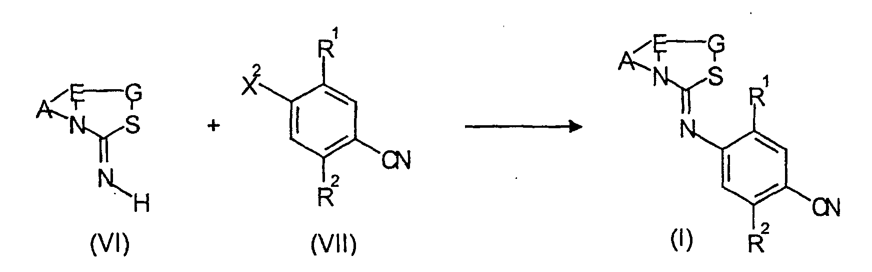

Die neuen 4-Cyanophenyliminoheterocyclen der allgemeinen Formel (I) zeichnen sich durch starke und selektive herbizide Wirksamkeit aus.

In den Definitionen sind die gesättigten oder ungesättigten Kohlenwasserstoffketten, wie Alkyl, Alkandiyl, Alkenyl, Alkendiyl oder Alkinyl, jeweils geradkettig oder verzweigt.

Halogen steht im allgemeinen für Fluor, Chlor, Brom oder Iod, vorzugsweise für Fluor, Chlor oder Brom, insbesondere für Fluor oder Chlor.

Gegenstand der Erfindung sind vorzugsweise Verbindungen der Formel (I), in welcher
- R¹: für Wasserstoff, Fluor oder Chlor steht,
- R²: für -N(R⁴)-SO₂R⁵ steht, wobei
- R⁴: für Wasserstoff, Methyl, Ethyl, n- oder i-Propyl, n-, i- oder s-Butyl steht,
- R⁵: für jeweils gegebenenfalls durch Fluor, Chlor, Brom, Cyano, Carboxy, Methoxy, Ethoxy, Methoxyethoxy, Ethoxyethoxy, Methylthio, Ethylthio, Acetyl, Propionyl, Methoxycarbonyl oder Ethoxycarbonyl substituiertes Methyl, Ethyl, n- oder i-Propyl, n-, i-, s- oder t-Butyl steht,
- R⁵: weiterhin für jeweils gegebenenfalls durch Fluor oder Chlor substituiertes Allyl, Crotonyl, 1-Methyl-allyl, Propargyl oder 1-Methyl-propargyl steht,
- R⁵: weiterhin für jeweils gegebenenfalls durch Fluor, Chlor, Methyl, Ethyl, n- oder i-Propyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl steht,
- R⁵: weiterhin für jeweils gegebenenfalls durch Fluor, Chlor, Brom, Cyano, Methyl, Ethyl, n- oder i-Propyl, n-, i-, s- oder t-Butyl, Methoxy, Ethoxy, Methylthio, Ethylthio, Methylsulfinyl, Ethylsulfinyl, Methylsulfonyl, Ethyl-sulfonyl, Trifluormethyl, Difluormethoxy, Trifluormethoxy, Trifluormethylthio, Trifluormethylsulfinyl, Trifluormethylsulfonyl, Dimethylaminosulfonyl, Methoxycarbonyl, Ethoxycarbonyl, Phenyl, Phenoxy oder Phenylsulfonyl substituiertes Phenyl, Naphthyl, Benzyl oder Phenylethyl steht,
- A: für Butan-1,4-diyl (Tetramethylen), 1-Buten-1,4-diyl oder 2-Buten-1,4-diyl steht,
- E: für Stickstoff steht und
- G: für Kohlenstoff steht, welcher über eine exo-Doppelbindung mit Sauerstoff verbunden ist.

Eine besonders bevorzugte Gruppe von Verbindungen der Formel (I) sind die Verbindungen der allgemeinen Formel (IB) 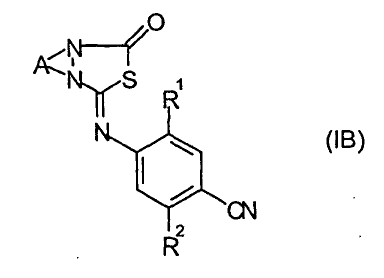 in welcher
- R¹, R² und A: die oben als bevorzugt angegebene Bedeutung haben.

Beispiele für die erfindungsgemäßen Verbindungen der Formel (I) sind in der nachstehenden Tabelle 1 aufgeführt.

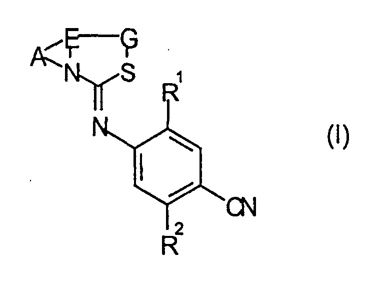

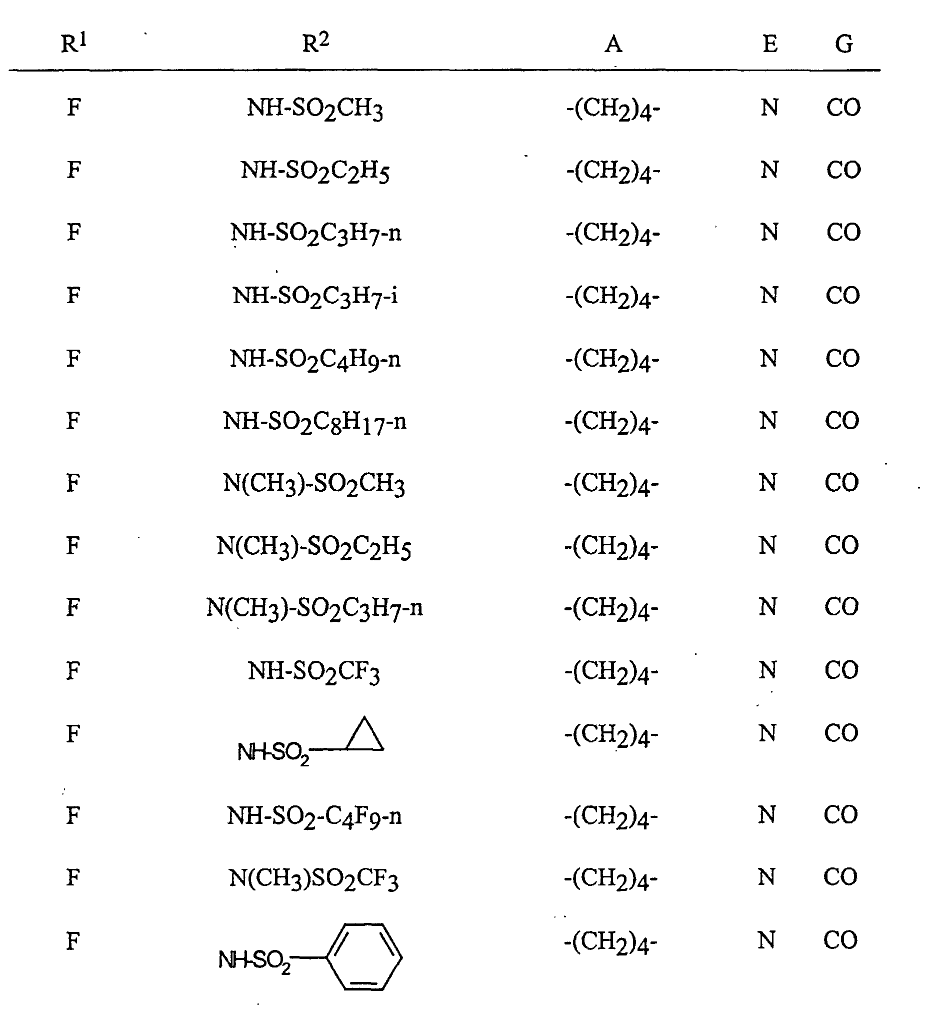

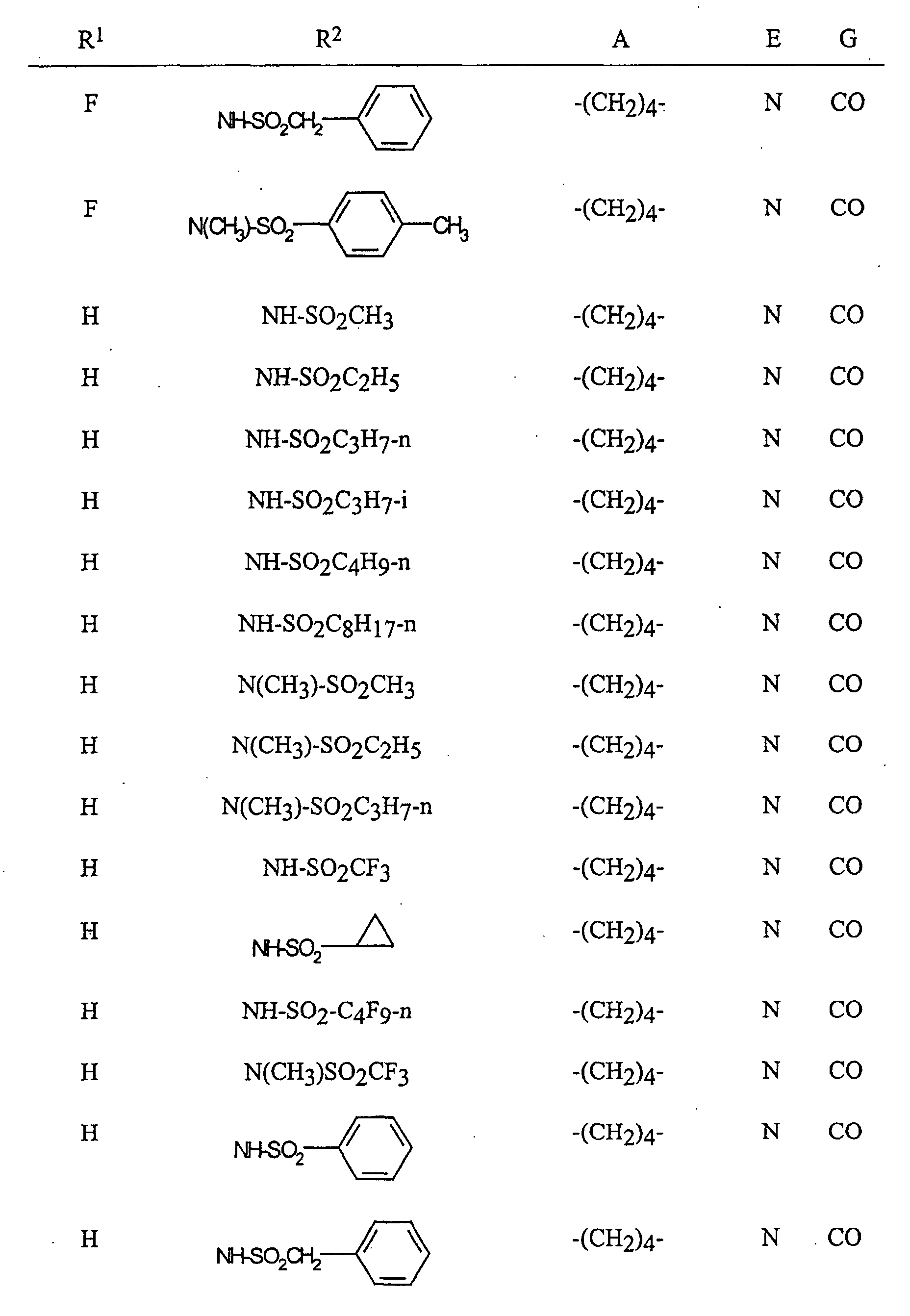

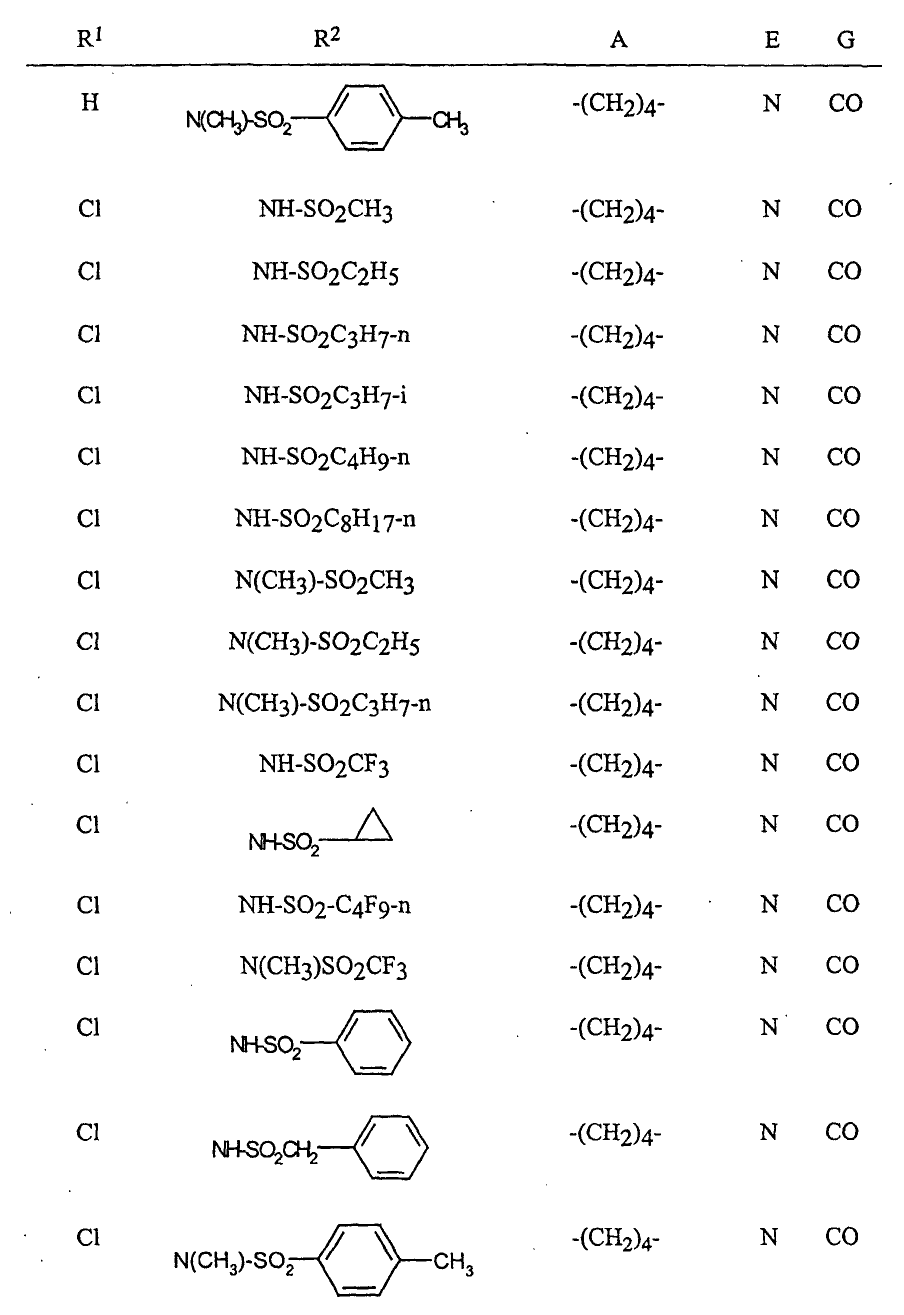

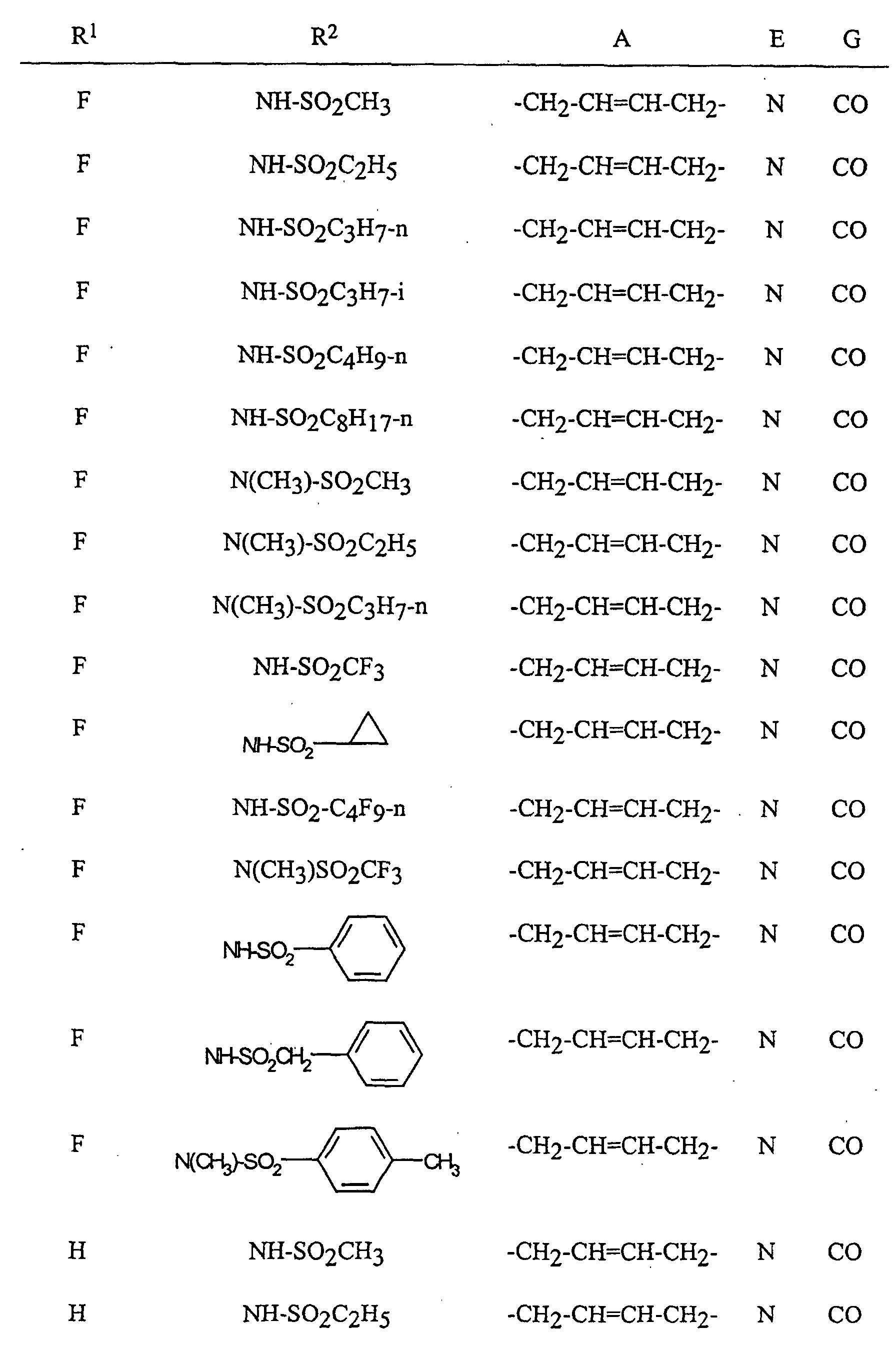

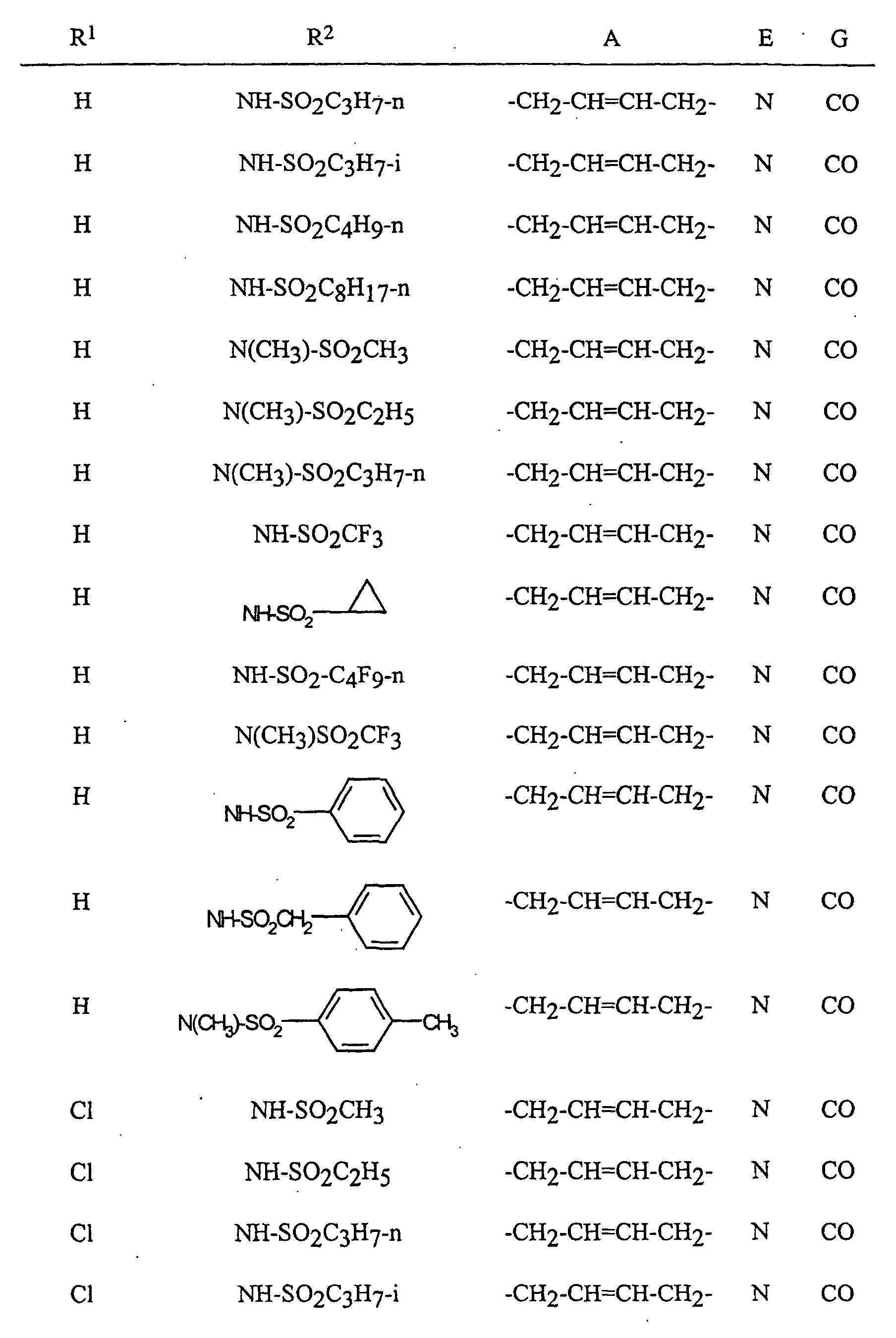

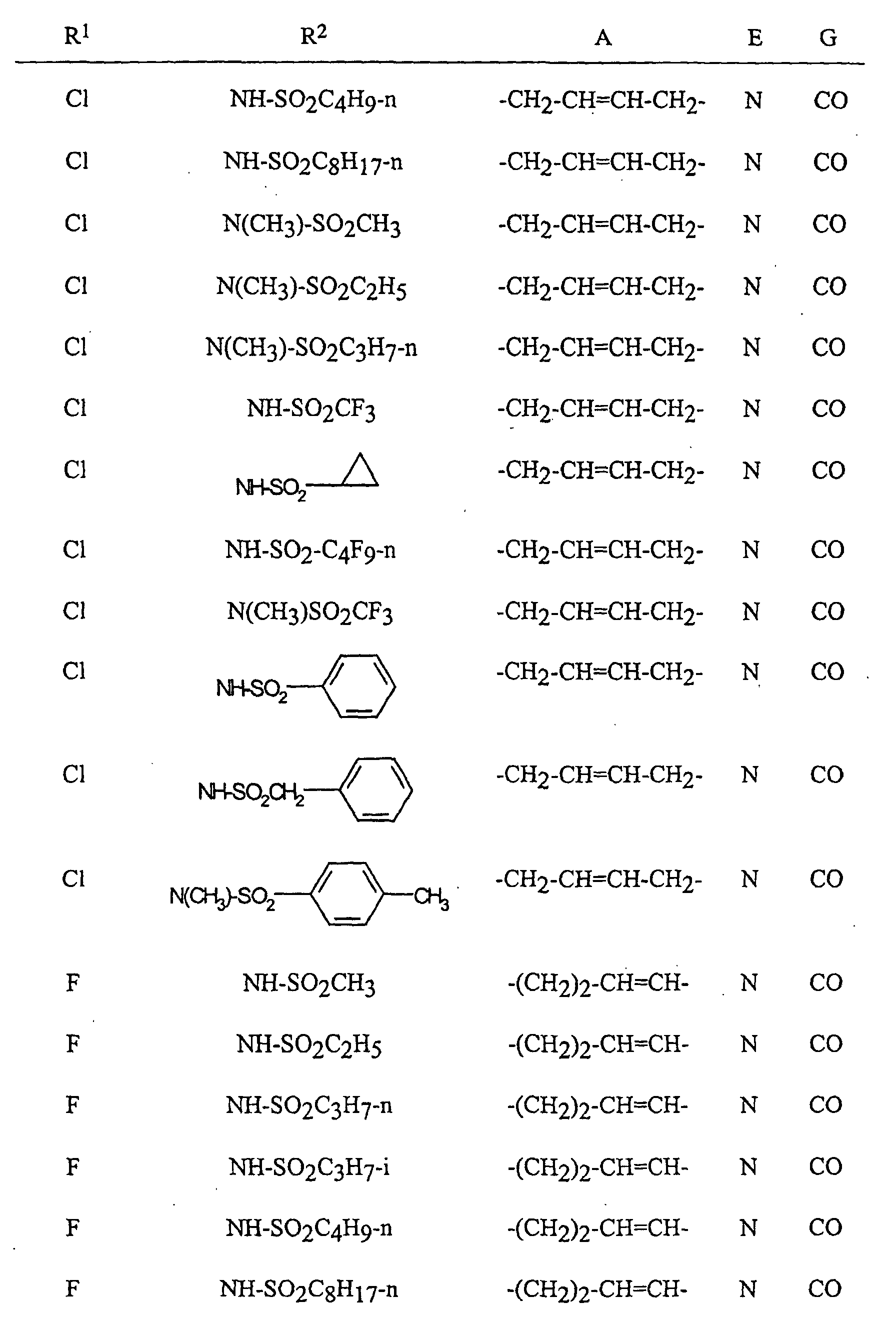

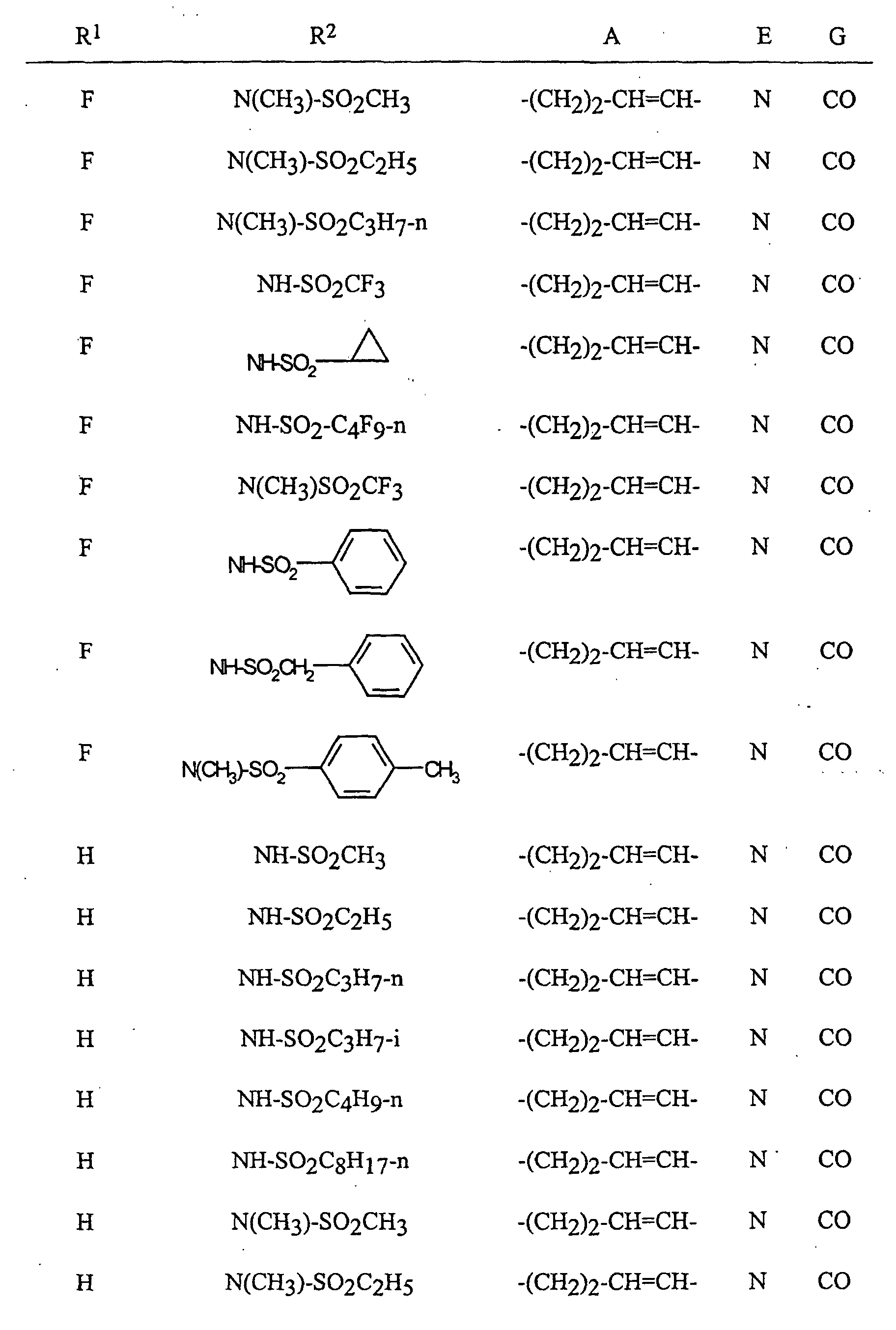

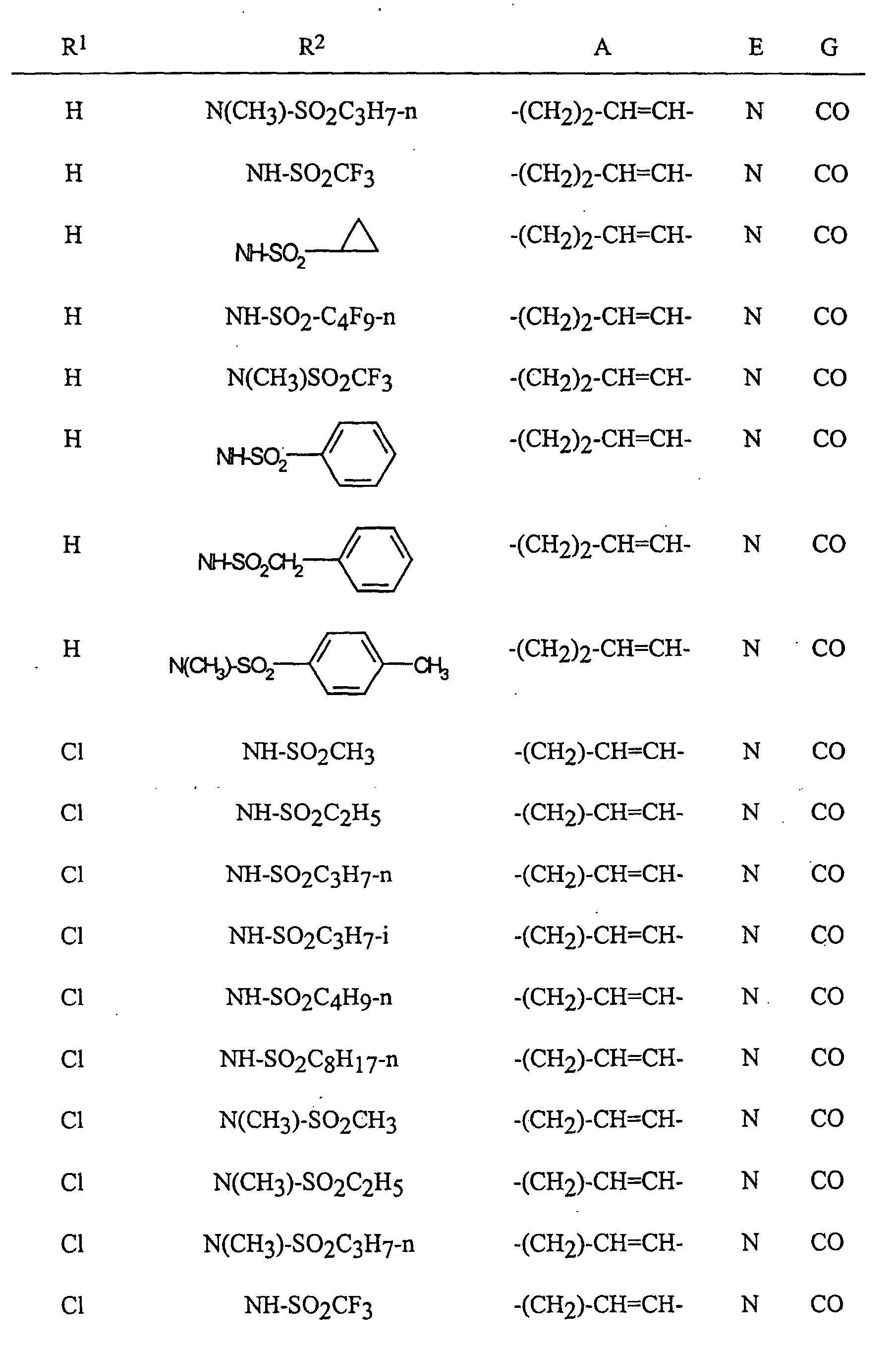

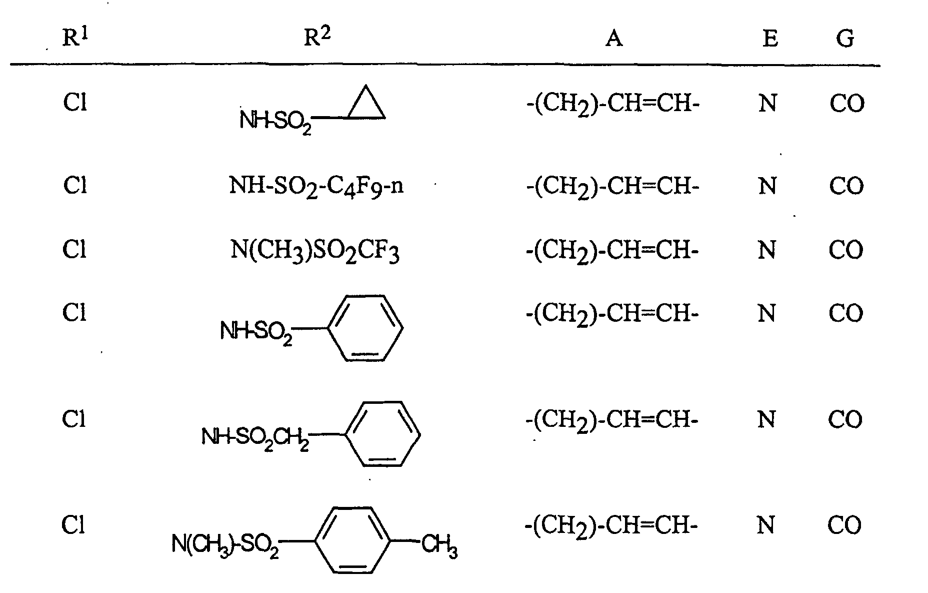

Die oben aufgeführten allgemeinen oder in Vorzugsbereichen angegebenen Restedefinitionen gelten sowohl für die Endprodukte der Formel (I), als auch entsprechend für die jeweils zu Herstellung benötigten Ausgangsstoffe bzw. Zwischenprodukte.

Diese Restedefinitionen können untereinander, also auch zwischen den angegebenen Bereichen bevorzugter Verbindungen, beliebig kombiniert werden.

Verwendet man beispielsweise 1-[N-(4-Cyano-2-chlor-5-ethoxy-phenyl)]-tetrahydro-(2H)-pyridazin-thiocarboxamid und Phosgen als Ausgangsstoffe, so kann der Reaktionsablauf beim erfindungsgemäßen Verfahren (a) durch das folgende Formelschema skizziert werden:

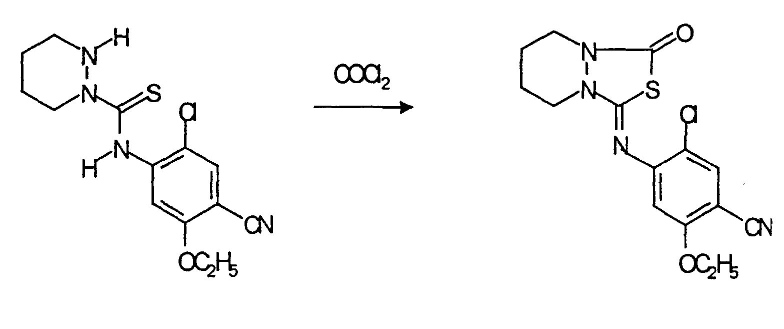

Verwendet man beispielsweise 9-(4-Cyano-2,5-difluor-phenyl-imino)-8-thia-1,6-diazabicyclo[4.3.0]nonan-7-on und Methansulfonamid als Ausgangsstoffe, so kann der Reaktionsablauf beim erfindungsgemäßen Verfahren (b) durch das folgende Formelschema skizziert werden:

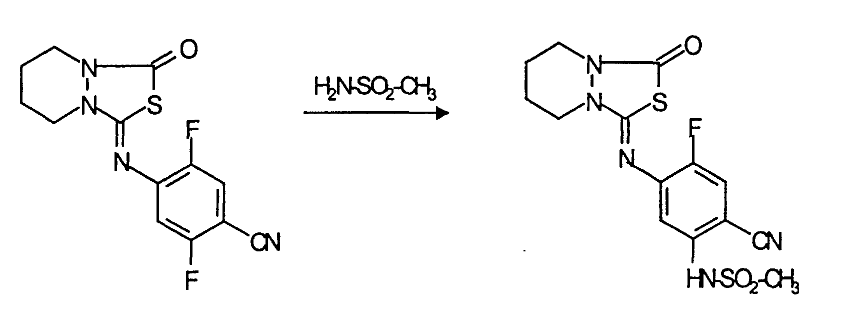

Die beim erfindungsgemäßen Verfahren (a) zur Herstellung der Verbindungen der Formel (I) als Ausgangsstoffe zu verwendenden substituierten Thiocarbonylaminoverbindungen sind durch die Formel (III) allgemein definiert.

In der Formel (III) haben R¹, R² und A vorzugsweise bzw. insbesondere diejenigen Bedeutungen, die bereits oben im Zusammenhang mit der Beschreibung der erfindungsgemäßen Verbindungen der Formel (I) als bevorzugt für R¹, R² und A angegeben wurden.

Die als Ausgangsstoffe benötigten substituierten Thiocarbonylaminoverbindungen der Formel (III) sind noch nicht aus der Literatur bekannt; sie sind als neue Stoffe Gegenstand der vorliegenden Anmeldung.

Man erhält die neuen Verbindungen der Formel (III), wenn man Diazacycloalkane bzw. Diazacycloalkene der allgemeinen Formel (XI) 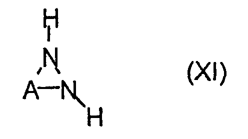 in welcher
- A: die oben angegebenen Bedeutung hat,
mit Cyanoarylisothiocyanaten der allgemeinen Formel (VIII) 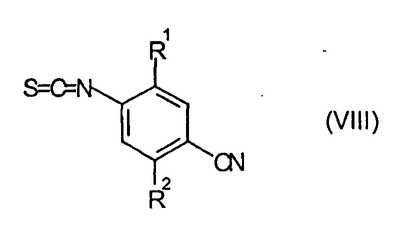 in welcher
- R¹ und R²: die oben angegebenen Bedeutungen haben,
gegebenenfalls in Gegenwart eines Verdünnungsmittels, wie z.B. Toluol, bei Temperaturen zwischen 0°C und 100°C umsetzt (vgl. die Herstellungsbeispiele).

Man erhält die neuen Cyanoarylisothiocyanate der Formel (VIII), wenn man entsprechende Cyanoarylamine der allgemeinen Formel (X) 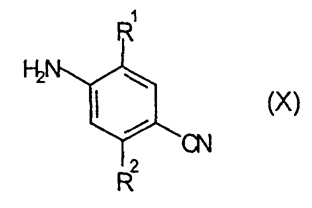 in welcher
- R¹ und R²: die oben angegebenen Bedeutungen haben,
mit Thiophosgen, gegebenenfalls in Gegenwart eines Reaktionshilfsmittels, wie z.B. Calciumcarbonat, und gegebenenfalls in Gegenwart von Verdünnungsmitteln, wie z.B. Methylenchlorid und Wasser, bei Temperaturen zwischen 0°C und 100°C umsetzt (vgl. die Herstellungsbeispiele).

Die Cyanoarylamine der allgemeinen Formel (X) sind bekannt und/oder können nach an sich bekannten Verfahren hergestellt werden (vgl. EP-A 224001).

Noch nicht aus der Literatur bekannt sind die Cyanoarylamine der allgemeinen Formel (Xa) 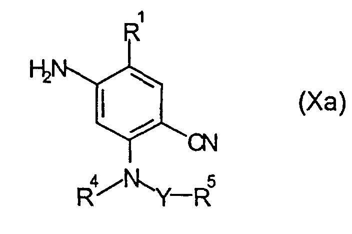 in welcher
- R¹, R⁴ und R⁵: die oben angegebenen Bedeutungen haben und
- Y: für CO oder SO₂ steht.
Man erhält die neuen Cyanoarylamine der Formel (Xa), wenn man entsprechende Halogenarylamine der allgemeinen Formel (X) 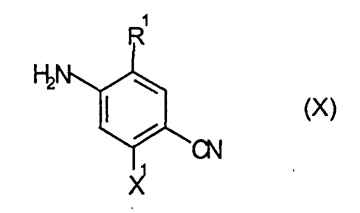 in welcher
- R¹ und X¹: die oben angegebenen Bedeutungen haben,
mit Amiden der allgemeinen Formel (XII)

R⁴-NH-Y-R⁵ (XII)

in welcher
- R⁴, R⁵ und Y: die oben angegebenen Bedeutungen haben,
gegebenenfalls in Gegenwart eines Säureakzeptors, wie z.B. Kaliumcarbonat, und gegebenenfalls in Gegenwart eines Verdünnungsmittels, wie z.B. N-Methyl-pyrrolidon, bei Temperaturen zwischen 100°C und 200°C umsetzt (vgl. die Herstellungsbeispiele).

Die als Vorprodukt benötigten Diazacycloalkane bzw. Diazacycloalkene der Formel (XI) sind bekannt und/oder können nach an sich bekannten Verfahren hergestellt werden (vgl. J. Org. Chem. 35 (1970), 1468-1471; Tetrahedron 31 (1975), 165-170).

Das erfindungsgemäße Verfahren (a) wird vorzugsweise unter Verwendung eines Verdünnungsmittels durchgeführt. Als Verdünnungsmittel kommen alle üblichen inerten organischen Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid.

Die Reaktionstemperaturen können bei der Durchführung des erfindungsgemäßen Verfahrens (a) in einem größeren Bereich variiert werden. Im allgemeinen arbeitet man bei Temperaturen zwischen 0°C und 150°C, vorzugsweise bei Temperaturen zwischen 10°C und 100°C.

Das erfindungsgemäße Verfahren (a) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (a) werden die jeweils benötigten Ausgangsstoffe im allgemeinen in angenähert äquimolaren bzw. äquivalenten Mengen eingesetzt. Es ist jedoch auch möglich, eine der jeweils eingesetzten Komponenten in einem größeren Überschuß zu verwenden. Die Umsetzung wird im allgemeinen in einem geeigneten Verdünnungsmittel durchgeführt und das Reaktionsgemisch wird bis zum Ende der Umsetzung bei der jeweils erforderlichen Temperatur gerührt. Die Aufarbeitung erfolgt nach üblichen Methoden (vgl. die Herstellungsbeispiele).

Die beim erfindungsgemäßen Verfahren (b) als Ausgangsstoffe zu verwendenden substituierten Cyanoaryliminoheterocyclen sind durch die Formel (Ia) allgemein definiert.

In der Formel (Ia) haben R¹, A, E und G vorzugsweise bzw. insbesondere diejenigen Bedeutungen, die bereits oben im Zusammenhang mit der Beschreibung der erfindungsgemäßen Verbindungen der Formel (I) als bevorzugt für R¹, A, E und G angegeben wurden;
- X¹: steht vorzugsweise für Fluor oder Chlor, insbesondere für Fluor.

Die Ausgangsstoffe der Formel (Ia) sind erfindungsgemäße, neue Verbindungen; sie können nach dem erfindungsgemäßen Verfahren (a) hergestellt werden.

Das erfindungsgemäße Verfahren (b) wird vorzugsweise unter Verwendung eines Verdünnungsmittels durchgeführt. Als Verdünnungsmittel kommen alle üblichen organischen oder anorganischen Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid oder Alkohole, wie Methanol, Ethanol, n-oder i-Propanol oder n-, i-, s- oder t-Butanol sowie Wasser.

Das erfindungsgemäße Verfahren (b) wird gegebenenfalls in Gegenwart eines Säureakzeptors durchgeführt. Als solche kommen vor allem die üblichen anorganischen oder organischen Basen in Betracht. Hierzu gehören beispielsweise Erdalkali- oder Alkalimetallhydroxide, wie Natriumhydroxid, Kaliumhydroxid, Calciumhydroxid oder auch Ammoniumhydroxid, Alkalimetall(hydrogen)carbonate, wie Natrium-(hydrogen)carbonat, Kalium(hydrogen)carbonat oder Ammoniumcarbonat, Alkalioder Erdalkalimetallacetate, wie Natriumacetat, Kaliumacetat, Calciumacetat oder Ammoniumacetat, Alkalimetall-alkoholate, wie Natrium- oder Kalium-methylat, Natrium- oder Kalium-ethylat, Natrium- oder Kalium-tert-butylat, sowie tertiäre Amine, wie Trimethylamin, Triethylamin, Tributylamin, N,N-Dimethylanilin, Pyridin, N-Methylpiperidin, 4-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (b) in einem größeren Bereich variiert werden. Im allgemeinen arbeitet man bei Temperaturen zwischen 0°C und 150°C, vorzugsweise bei Temperaturen zwischen 10°C und 120°C.

Das erfindungsgemäße Verfahren (b) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (b) werden die jeweils benötigten Ausgangsstoffe im allgemeinen in angenähert äquimolaren bzw. äquivalenten Mengen eingesetzt. Es ist jedoch auch möglich, eine der jeweils eingesetzten Komponenten in einem größeren Überschuß zu verwenden. Die Umsetzung wird im allgemeinen in einem geeigneten Verdünnungsmittel durchgeführt und das Reaktionsgemisch wird bis zum Ende der Umsetzung bei der jeweils erforderlichen Temperatur gerührt. Die Aufarbeitung erfolgt nach üblichen Methoden.

Die erfindungsgemäßen Wirkstoffe können als Defoliants, Desiccants, Krautabtötungsmittel und insbesondere als Unkrautvernichtungsmittel verwendet werden.Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewandten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.
Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Aperä.
Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Zitrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen und dikotylen Unkräutern in monokotylen Kulturen sowohl im Vorauflauf- als auch im Nachauflauf-Verfahren.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, Alkylnaphthaline, chlorierte Aromaten oder chlorierte aliphatische Kohlenwasserstoffe, wie Chlorbenzole, Chlorethylene, oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid oder Dimethylsulfoxid, sowie Wasser.

Als feste Trägerstoffe kommen infrage: z.B. Ammoniumsalze und natürliche Gesteinsmehle, wie Kaoline, Tonerden, Talkum, Kreide, Quartz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infrage: z.B. nicht ionogene und anionische Emulgatoren, wie Polyoxyethylen-Fettsäureester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen infrage: z.B. Ligninsulfitablaugen und Methylcellulose.

Es können in den Formulierungen Haftmittel wie Carboxymethylcellulose, natürliche und synthetische, pulverige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinenzwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba oder Picloram; Aryloxyalkansäuren, wie z.B. 2,4-D, 2,4-DB, 2,4-DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen; Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,001 und 10 kg Wirkstoff pro Hektar Bodenfläche, vorzugsweise zwischen 0,005 und 5 kg pro Hektar.

Die Herstellung und die Verwendung der erfindungsgemäßen Wirkstoffe geht aus den nachfolgenden Beispielen hervor.

Die Herstellung und die Verwendung der erfindungsgemäßen Wirkstoffe geht aus den nachfolgenden Beispielen hervor.

### Herstellungsbeispiele:

### Beispiel 1

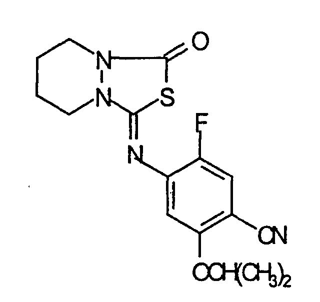

Zu einer Lösung von 9,7 g (0,03 Mol) 1-[N-(4-Cyano-2-fluor-5-i-propoxy-phenyl)]-tetrahydro-(2H)-pyridazin-thiocarboxamid in 150 ml Dichlormethan gibt man tropfenweise bei 20°C 26 ml (0,05 Mol) einer 20%igen Lösung von Phosgen in Toluol. Die Reaktionsmischung wird 3 Stunden bei 20°C gerührt und dann auf etwa die gleiche Menge Eiswasser gegeben. Die organische Phase wird abgetrennt, mit Magnesiumsulfat getrocknet und filtriert. Das Filtrat wird im Wasserstrahlvakuum eingeengt und der Rückstand durch Säulenchromatographie (Kieselgel, Dichlormethan) gereinigt.

Man erhält 1,4 g (13 % der Theorie) 9-(4-Cyano-2-fluor-5-isopropoxy-phenylimino)-8-thia-1,6-diazabicyclo[4.3.0]nonan-7-on vom Schmelzpunkt 108°C.

Analog Beispiel 1 sowie entsprechend der allgemeinen Beschreibung der erfindungsgemäßen Herstellungsverfahren können beispielsweise auch die in der nachstehenden Tabelle 2 aufgeführten Verbindungen der Formel (I) hergestellt werden.

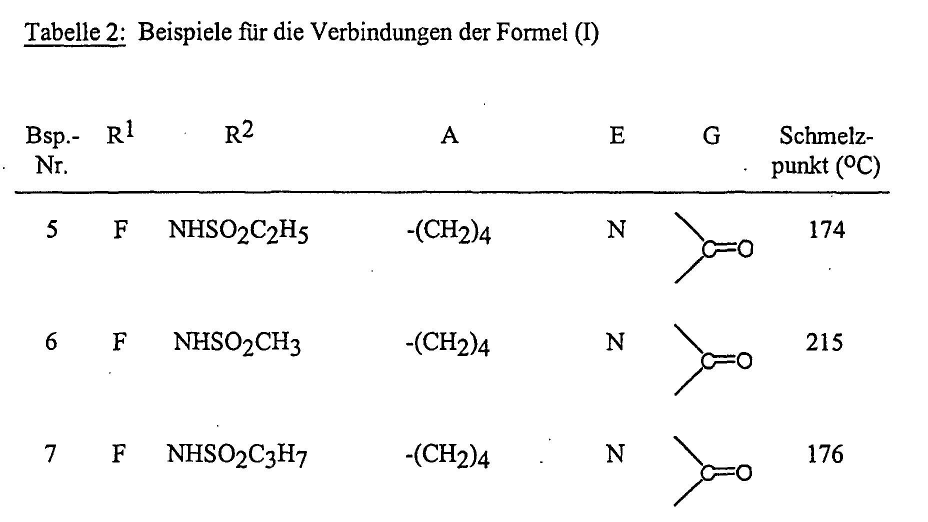

### Ausgangsstoffe der Formel (III):

### Beispiel (III-1)

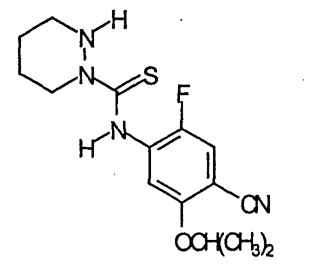

Zu einer Lösung von 2,9 g (34 mMol) Hexahydropyridazin in 40 ml Toluol gibt man unter Rühren bei 20°C 8,0 g (34 mMol) 4-Cyano-2-fluor-5-isopropoxy-phenylisothiocyanat. Das Reaktionsgemisch wird 16 Stunden bei 20°C gerührt und anschließend im Wasserstrahlvakuum eingeengt.

Man erhält 10,3 g (94 % der Theorie) 1-[N-(4-Cyano-2-fluor-5-isopropoxy-phenyl)]-tetrahydro-(2H)-pyridazin-thiocarboxamid als öligen Rückstand, welcher allmählich kristallin erstarrt.
Schmelzpunkt: 42°C.

**Tabelle 3:**

| Beispiele für die Verbindungen der Formel (III) | | | | |
|---|---|---|---|---|
| Bsp.-Nr. | R¹ | R² | A | Schmelzpunkt (°C) |
| III-2 | F | NHSO₂CH₃ | -(CH₂)₄- | |

### Ausgangsstoffe der Formel (VIII):

### Beispiel (VIII-1)

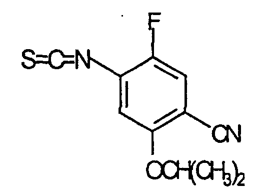

Zu einer Mischung aus 6 g (0,06 Mol) Calciumcarbonat, 30 ml Wasser, 6,9 g (0,06 Mol) Thiophosgen und 30 ml Dichlormethan gibt man unter Rühren bei ca. 30°C 7,8 g (0,04 Mol) 4-Cyano-2-fluor-5-isopropoxy-anilin in 25 ml Dichlormethan. Das Reaktionsgemisch wird ca. 18 Stunden bei 30°C bis 35°C gerührt. Dann wird nach Filtration die organische Phase abgetrennt, mit Magnesiumsulfat getrocknet und filtriert. Vom Filtrat wird das Lösungsmittel im Wasserstrahlvakuum sorgfältig abdestilliert.

Man erhält 8,4 g (89 % der Theorie) 4-Cyano-2-fluor-5-isopropoxy-phenyl-isothiocyanat als öligen Rückstand, welcher allmählich kristallin erstarrt.
Schmelzpunkt; 73°C.

**Tabelle 4:**

| Beispiele für die Verbindungen der Formel (VIII) | | | |
|---|---|---|---|
| Bsp.-Nr. | R¹ | R² | Schmelzpunkt (°C) |
| (VIII-2) | F | NHSO₂CH₃ | 166 |

### Ausgangsstoffe der Formel (X):

### Beispiel X-1:

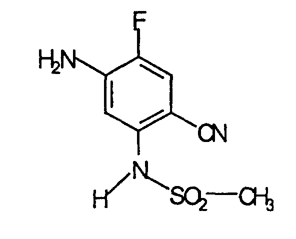

Eine Mischung aus 92,4 g (0,6 Mol) 4-Cyano-2,5-difluor-anilin, 60 g (0,60 Mol) Methansulfonamid, 166 g Kaliumcarbonat und 80 ml N-Methyl-pyrrolidon wird 10 Stunden auf 180°C erhitzt. Nach dem Erkalten wird die Mischung in 5 Liter Wasser eingerührt und die erhaltene Lösung zweimal mit je 400 ml Essigsäureethylester gewaschen. Die wäßrige Phase wird dann mit 300 ml Essigsäureethylester überschichtet und mit 10%iger Salzsäure angesäuert. Das kristallin anfallende Produkt wird dann durch Absaugen isoliert.

Man erhält 70 g (51% der Theorie) N-(5-Amino-2-cyano-4-fluor-phenyl)-methansulfonamid vom Schmelzpunkt 238°C.

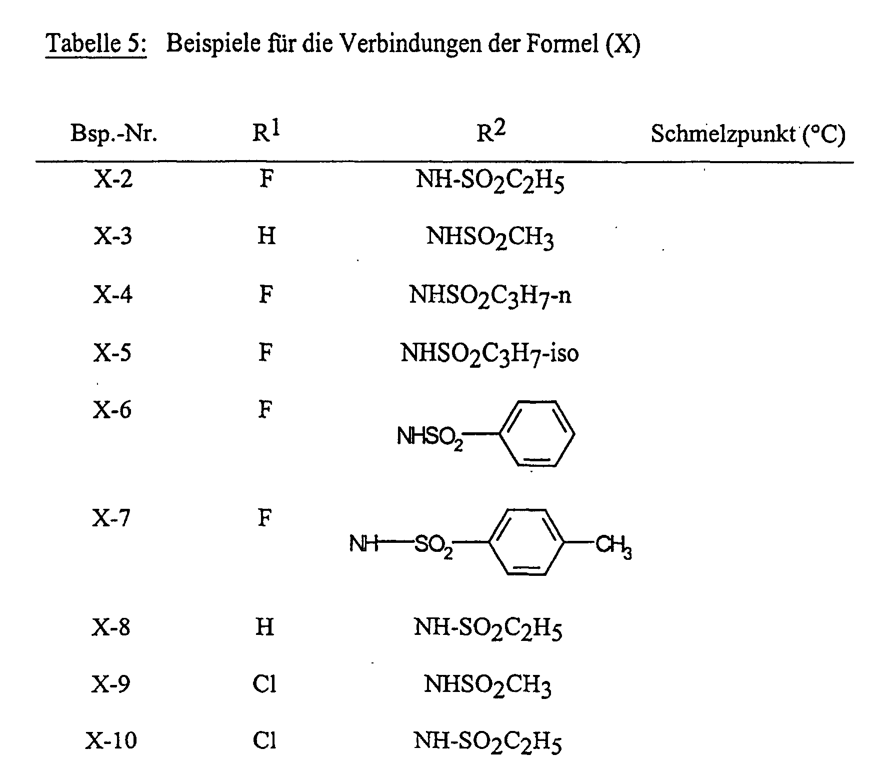

### Anwendungsbeispiele:

### Beispiel A

### Post-emergence-Test

| | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Nach drei Wochen wird der Schädigungsgrad der Pflanzen bonitiert in % Schädigung im Vergleich zur Entwicklung der unbehandelten Kontrolle.

Es bedeuten:

| | |
|---|---|
| O % = | keine Wirkung (wie unbehandelte Kontrolle) |
| 100 % = | totale Vernichtung |

In diesem Test zeigt beispielsweise die Verbindung gemäß Herstellungsbeispiel 1 bei einer Aufwandmenge von 15 g/ha und sehr guter Verträglichkeit gegenüber Kulturpflanzen, wie z.B. Gerste (0 %), starke Wirkung gegen Unkräuter wie Abutilon (100 %), Amaranthus (80 %), Chenopodium (95 %), Ipomoea (100 %) und Veronica (85 %).

### Beispiel B

### Pre-emergence-Test

| | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle; entscheidend ist nur die Aufwandmenge des Wirkstoffs pro Flächeneinheit.

Nach drei Wochen wird der Schädigungsgrad der Pflanzen bonitiert in % Schädigung im Vergleich zur Entwicklung der unbehandelten Kontrolle.

Es bedeuten:

| | |
|---|---|
| O % = | keine Wirkung (wie unbehandelte Kontrolle) |
| 100 % = | totale Vernichtung |

In diesem Test zeigt beispielsweise die Verbindung gemäß Herstellungsbeispiel 1 bei einer Aufwandmenge von 60 g/ha und sehr guter Verträglichkeit gegenüber Kulturpflanzen, wie z.B. Gerste (0 %), starke Wirkung gegen Unkräuter wie Aeopecurus (90 %), Digiteria (95 %), Abutilon 100 %), Chenopodium 100 %), Matricaria (100 %) und Dinapis (80 %).

## Patentansprüche

1. 4-Cyanophenyliminoheterocyclen der allgemeinen Formel (I) 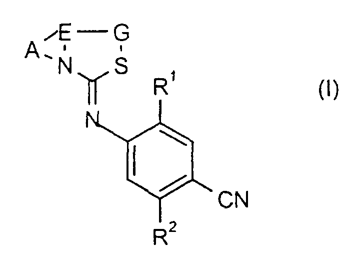 in welcher
R¹ für Wasserstoff, Fluor, Chlor oder Brom steht,
R² für -N(R⁴)-SO₂R⁵ steht,
R⁴ für Wasserstoff oder für Alkyl mit 1 bis 8 Kohlenstoffatomen steht und
R⁵ für gegebenenfalls durch Fluor, Chlor, Brom, Cyano, Carboxy, C₁-C₄-Alkoxy, C₁-C₄-Alkoxy-C₁-C₄-alkoxy, C₁-C₄-Alkylthio, C₁-C₄-Alkyl-carbonyl oder C₁-C₄-Alkoxy-carbonyl substituiertes Alkyl mit 1 bis 8 Kohlenstoffatomen steht,
R⁵ weiterhin für jeweils gegebenenfalls durch Fluor, Chlor oder Brom substituiertes Alkenyl oder Alkinyl mit jeweils 2 bis 8 Kohlenstoffatomen steht,
R⁵ weiterhin für jeweils gegebenenfalls durch Fluor, Chlor, Brom, Cyano, Carboxy, C₁-C₄-Alkyl oder C₁-C₄-Alkoxy-carbonyl substituiertes Cycloalkyl oder Cycloalkylalkyl mit jeweils 3 bis 8 Kohlenstoffatomen im Cycloalkylteil und gegebenenfalls 1 bis 4 Kohlenstoffatomen im Alkylteil oder für jeweils gegebenenfalls durch Fluor, Chlor, Brom, Cyano, Nitro, Carboxy, Carbamoyl, durch C₁-C₄-Alkyl, C₁-C₄-Alkoxy, C₁-C₄-Alkylthio, C₁-C₄-Alkylsulfinyl oder C₁-C₄-Alkylsulfonyl (welche jeweils gegebenenfalls durch Fluor und/oder Chlor substituiert sind), durch Dimethylaminosulfonyl oder Diethylaminosulfonyl, durch C₁-C₄-Alkoxy-carbonyl (welches gegebenenfalls durch Fluor, Chlor, Brom, Methoxy oder Ethoxy substituiert ist), durch Phenyl, Phenylmethyl, Phenoxy, Phenylthio, Phenylsulfinyl oder Phenylsulfonyl (welche jeweils gegebenenfalls durch Fluor, Chlor, Brom, Cyano, Methyl, Methoxy, Trifluormethyl, Difluormethoxy und/oder Trifluormethoxy substituiert sind) substituiertes Aryl oder Arylalkyl mit 6 oder 10 Kohlenstoffatomen im Arylteil und gegebenenfalls 1 bis 4 Kohlenstoffatomen im Alkylteil steht,
A für Alkandiyl oder Alkendiyl mit jeweils 4 Kohlenstoffatomen steht,
E für Stickstoff steht und
G für Kohlenstoff steht, welcher über eine exo-Doppelbindung mit Sauerstoff verbunden ist,

2. 4-Cyanophenyliminoheterocyclen der allgemeinen Formel (I) gemäß Anspruch 1, **dadurch gekennzeichnet, daß**
R¹ für Wasserstoff, Fluor oder Chlor steht,
R² für -N(R⁴)-SO₂R⁵ steht,
R⁴ für Wasserstoff, Methyl, Ethyl, n- oder i-Propyl, n-, i- oder s-Butyl steht,
R⁵ für jeweils gegebenenfalls durch Fluor, Chlor, Brom, Cyano, Carboxy, Methoxy, Ethoxy, Methoxyethoxy, Ethoxyethoxy, Methylthio, Ethylthio, Acetyl, Propionyl, Methoxycarbonyl oder Ethoxycarbonyl substituiertes Methyl, Ethyl, n- oder i-Propyl, n-, i-, s- oder t-Butyl steht,
R⁵ weiterhin für jeweils gegebenenfalls durch Fluor oder Chlor substituiertes Allyl, Crotonyl, 1-Methyl-allyl, Propargyl oder 1-Methylpropargyl steht,
R⁵ weiterhin für jeweils gegebenenfalls durch Fluor, Chlor, Methyl, Ethyl, n- oder i-Propyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl steht,
R⁵ weiterhin für jeweils gegebenenfalls durch Fluor, Chlor, Brom, Cyano, Methyl, Ethyl, n- oder i-Propyl, n-, i-, s- oder t-Butyl, Methoxy, Ethoxy, Methylthio, Ethylthio, Methylsulfinyl, Ethylsulfinyl, Methylsulfonyl, Ethylsulfonyl, Trifluormethyl, Difluormethoxy, Trifluormethoxy, Trifluormethylthio, Trifluormethylsulfinyl, Trifluormethylsulfonyl, Dimethylaminosulfonyl, Methoxycarbonyl, Ethoxycarbonyl, Phenyl, Phenoxy oder Phenylsulfonyl substituiertes Phenyl, Naphthyl, Benzyl oder Phenylethyl steht,
A für Butan-1,4-diyl (Tetramethylen), 1-Buten-1,4-diyl oder 2-Buten-1,4-diyl steht,
E für Stickstoff steht und
G für Kohlenstoff steht, welcher über eine exo-Doppelbindung mit Sauerstoff verbunden ist.

3. 4-Cyanophenyliminoheterocyclen gemäß Anspruch 1, **gekennzeichnet durch** die allgemeine Formel (IB) 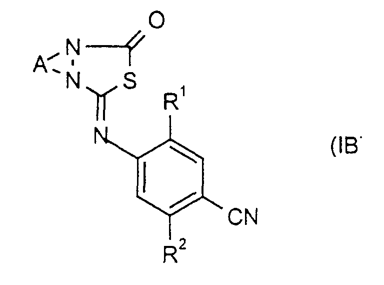 in welcher R¹, R² und A die in Anspruch 1 angegebenen Bedeutungen haben.

4. Verfahren zur Herstellung von 4-Cyanophenyliminoheterocyclen der allgemeinen Formel (I) gemäß Anspruch 1, **dadurch gekennzeichnet, daß** man
a) substituierte Thiocarbonylaminoverbindungen der allgemeinen Formel (III) 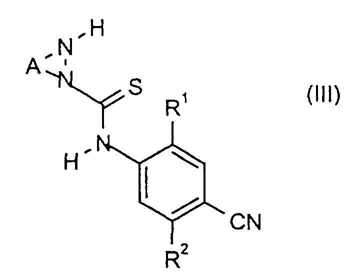 in welcher
A, R¹ und R² die in Anspruch 1 angegebenen Bedeutungen haben,
mit Phosgen, gegebenenfalls in Gegenwart eines Verdünnungsmittels umsetzt,
oder daß man
b) Cyanoaryliminoheterocyclen der allgemeinen Formel (Ia) 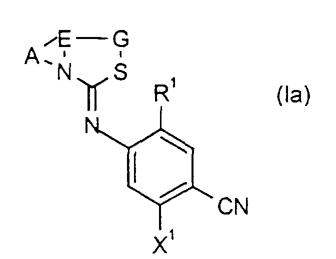
in welcher
R¹, A, E und G die in Anspruch 1 angegebenen Bedeutungen haben und
X¹ für Halogen steht,
mit nucleophilen Verbindungen der allgemeinen Formel (V)
H-N(R⁴)-SO₂R⁵ (V)
in welcher
R⁴ und R⁵ die in Anspruch 1 angegebenen Bedeutungen haben,
- oder mit Alkalimetallsalzen dieser Verbindungen -
gegebenenfalls in Gegenwart eines Säureakzeptors und gegebenenfalls in Gegenwart eines Verdünnungsmittels umsetzt.

5. Herbizide Mittel, **gekennzeichnet durch** einen Gehalt an mindestens einem 4-Cyanophenyliminoheterocyclus der Formel (I) gemäß einem der Ansprüche 1 bis 3.

6. Verfahren zur Bekämpfung von unerwünschten Pflanzen, **dadurch gekennzeichnet, daß** man 4-Cyanophenyliminoheterocyclen der allgemeinen Formel (I) gemäß einem der Ansprüche 1 bis 3 auf Pflanzen und/oder ihren Lebensraum einwirken läßt.

7. Verwendung von 4-Cyanophenyliminoheterocyclen der allgemeinen Formel (I) gemäß einem der Ansprüche 1 bis 3 zur Bekämpfung von unerwünschten Pflanzen.

8. Verfahren zur Herstellung von herbiziden Mitteln, **dadurch gekennzeichnet, daß** man 4-Cyanophenyliminoheterocyclen der allgemeinen Formel (I) gemäß einem der Ansprüche 1 bis 3 mit Streckmitteln und/oder oberflächenaktiven Substanzen vermischt.

9. Substituierte Thiocarbonylaminoverbindungen der allgemeinen Formel (III) 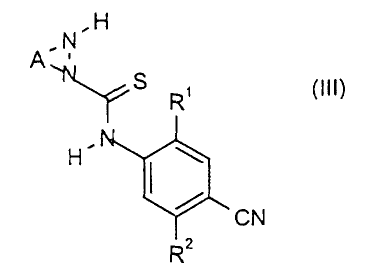 in welcher R¹, R² und A die in Anspruch 1 angegebenen Bedeutungen haben.

## Claims

1. 4-Cyanophenylimino heterocycles of the general formula (I) 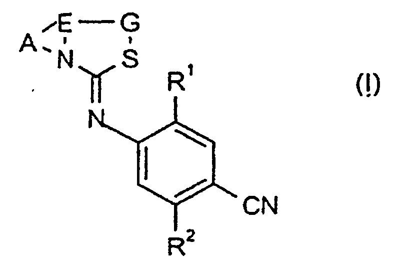 in which
R¹ represents hydrogen, fluorine, chlorine or bromine,
R² represents -N(R⁴)-SO₂R⁵,
R⁴ represents hydrogen or represents alkyl having 1 to 8 carbon atoms, and
R⁵ represents alkyl having 1 to 8 carbon atoms and which is optionally substituted by fluorine, chlorine, bromine, cyano, carboxyl, C₁-C₄-alkoxy, C₁-C₄-alkoxy-C₁-C₄-alkoxy, C₁-C₄-alkylthio, C₁-C₄-alkyl-carbonyl or C₁-C₄-alkoxy-carbonyl,
R⁵ furthermore represents alkenyl or alkinyl having in each case 2 to 8 carbon atoms and which are in each case optionally substituted by fluorine, chlorine or bromine,
R⁵ furthermore represents cycloalkyl or cycloalkyl-alkyl having in each case 3 to 8 carbon atoms in the cycloalkyl moiety and optionally 1 to 4 carbon atoms in the alkyl moiety and which are in each case optionally substituted by fluorine, chlorine, bromine, cyano, carboxyl, C₁-C₄-alkyl or C₁-C₄-alkoxy-carbonyl, or represents aryl or arylalkyl having 6 or 10 carbon atoms in the aryl moiety and optionally 1 to 4 carbon atoms in the alkyl moiety and which are in each case optionally substituted by fluorine, chlorine, bromine, cyano, nitro, carboxyl, carbamoyl, by C₁-C₄-alkyl, C₁-C₄-alkoxy, C₁-C₄-alkylthio, C₁-C₄-alkylsulphinyl or C₁-C₄-alkylsulphonyl (which are in each case optionally substituted by fluorine and/or chlorine), by dimethylaminosulphonyl or diethylaminosulphonyl, by C₁-C₄-alkoxy-carbonyl (which is optionally substituted by fluorine, chlorine, bromine, methoxy or ethoxy), by phenyl, phenylmethyl, phenoxy, phenylthio, phenylsulphinyl or phenylsulphonyl (which are in each case optionally substituted by fluorine, chlorine, bromine, cyano, methyl, methoxy, trifluoromethyl, difluoromethoxy and/or trifluoromethoxy),
A represents alkanediyl or alkenediyl having in each case 4 carbon atoms,
E represents nitrogen, and
G represents carbon which is bonded to oxygen via an exo double bond

2. 4-Cyanophenylimino heterocycles of the general formula (I) according to Claim 1, **characterized in that**
R¹ represents hydrogen, fluorine or chlorine,
R² represents -N(R⁴)-SO₂R⁵,
R⁴ represents hydrogen, methyl, ethyl, n- or i-propyl or n-, i- or s-butyl,
R⁵ represents methyl, ethyl, n- or i-propyl or n-, i-, s- or t-butyl which are in each case optionally substituted by fluorine, chlorine, bromine, cyano, carboxyl, methoxy, ethoxy, methoxyethoxy, ethoxyethoxy, methylthio, ethylthio, acetyl, propionyl, methoxycarbonyl or ethoxycarbonyl,
R⁵ furthermore represents allyl, crotonyl, 1-methyl-allyl, propargyl or 1-methyl-propargyl which are in each case optionally substituted by fluorine or chlorine,
R⁵ furthermore represents cyclopropyl, cyclobutyl, cyclopentyl, cyclohexyl, cyclopropylmethyl, cyclobutylmethyl, cyclopentylmethyl or cyclohexylmethyl which are in each case optionally substituted by fluorine, chlorine, methyl, ethyl or n- or i-propyl,
R⁵ furthermore represents phenyl, naphthyl, benzyl or phenylethyl which are in each case optionally substituted by fluorine, chlorine, bromine, cyano, methyl, ethyl, n- or i-propyl, n-, i-, s-or t-butyl, methoxy, ethoxy, methylthio, ethyl-thio, methylsulphinyl, ethylsulphinyl, methyl-sulphonyl, ethylsulphonyl, trifluoromethyl, difluoromethoxy, trifluoromethoxy, trifluoro-methylthio, trifluoromethylsulphinyl, trifluoro-methylsulphonyl, dimethylaminosulphonyl, methoxycarbonyl, ethoxycarbonyl, phenyl, phenoxy or phenylsulphonyl,
A represents butane-1,4-diyl (tetramethylene), 1-butene-1,4-diyl or 2-butene-1,4-diyl,
E represents nitrogen, and
G represents carbon which is bonded to oxygen via an exo double bond.

3. 4-Cyanophenylimino heterocycles according to Claim 1, **characterized by** the general formula (IB) 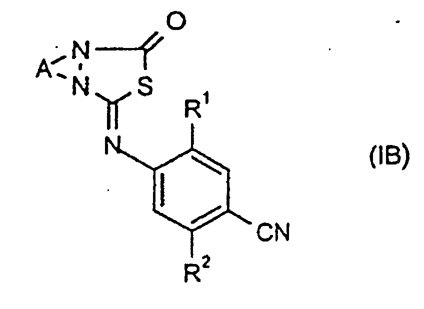 in which R¹, R² and A have the meanings mentioned in Claim 1.

4. Process for preparing 4-cyanophenylimino heterocycles of the general formula (I), according to Claim 1, **characterized in that**
a) substituted thiocarbonylamino compounds of the general formula (III) 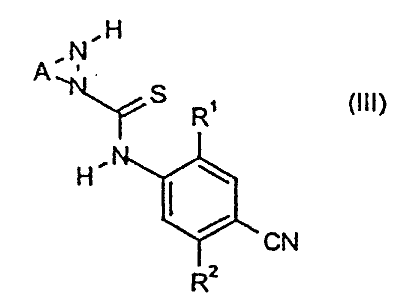 in which
A, R¹ and R² have the meanings mentioned in Claim 1,
are reacted with phosgene, optionally in the presence of a diluent,
or **in that**
b) cyanoarylimino heterocycles of the general formula (Ia) 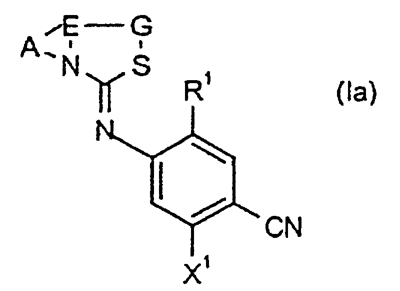 in which
R¹, A, E and G have the meanings mentioned in Claim 1, and
X¹ represents halogen,
are reacted with nucleophilic compounds of the general formula (V)
H-N(R⁴)-SO₂R⁵ (V)
in which
R⁴ and R⁵ have the meanings mentioned in Claim 1,
- or with alkali metal salts of these compounds -
optionally in the presence of an acid acceptor and optionally in the presence of a diluent.

5. Herbicidal agent, **characterized by** a content of at least one 4-cyanophenylimino heterocycle of the formula (I) according to one of Claims 1 to 3.

6. Process for controlling unwanted plants, **characterized in that** 4-cyanophenylimino heterocycles of the general formula (I) according to one of Claims 1 to 3 are allowed to act on plants and/or their habitat.

7. Use of 4-cyanophenylimino heterocycles of the general formula (I) according to one of Claims 1 to 3 for controlling unwanted plants.

8. Process for preparing herbicidal agents, **characterized in that** 4-cyanophenylimino heterocycles of the general formula (I) according to one of Claims 1 to 3 are mixed with extenders and/or surface-active substances.

9. Substituted thiocarbonylamino compounds of the general formula (III) 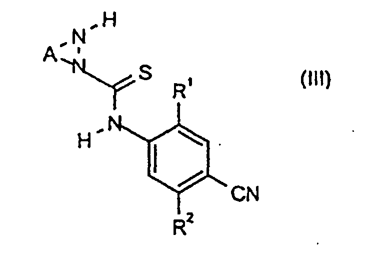 in which R¹, R² and A have the meanings mentioned in Claim 1.

## Revendications

1. 4-cyanophényliminohétérocycles de formule générale (I) 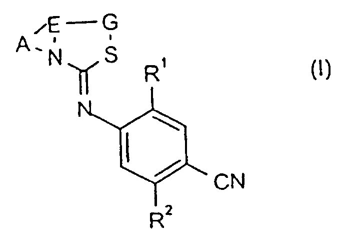 dans laquelle
R¹ représente l'hydrogène, le fluor, le chlore ou le brome,
R² est un groupe -N(R⁴)-SO₂R⁵,
R⁴ est l'hydrogène ou un groupe alkyle ayant 1 à 8 atomes de carbone et
R⁵ représente un groupe alkyle ayant 1 à 8 atomes de carbone, éventuellement substitué par du fluor, du chlore, du brome, un radical cyano, carboxy, alkoxy en C₁ à C₄, (alkoxy en C₁ à C₄) - (alkoxy en C₁ à C₄), alkyl-thio en C₁ à C₄, (alkyle en C₁ à C₄) -carbonyle ou (alkoxy en C₁ à C₄)-carbonyle,
R⁵ représente en outre un groupe alcényle ou un groupe alcynyle ayant chacun 2 à 8 atomes de carbone et chacun étant éventuellement substitué par du fluor, du chlore ou du brome,
R⁵ représente en outre un groupe cycloalkyle ou un groupe cycloalkylalkyle ayant chacun 3 à 8 atomes de carbone dans la partie cycloalkyle et, le cas échéant, 1 à 4 atomes de carbone dans la partie alkyle et chacun étant éventuellement substitué par du fluor, du chlore, du brome, un radical cyano, carboxy, alkyle en C₁ à C₄ ou (alkoxy en C₁ à C₄) -carbonyle, ou un groupe aryle ou arylalkyle ayant chacun 6 ou 10 atomes de carbone dans la partie aryle et, le cas échéant, 1 à 4 atomes de carbone dans la partie alkyle et chacun étant éventuellement substitué par du fluor, du chlore, du brome, un radical cyano, nitro, carboxy, carbamoyle, par un radical alkyle en C₁ à C₄, alkoxy en C₁ à C₄, alkylthio en C₁ à C₄, alkylsulfinyle en C₁ à C₄ ou alkylsulfonyle en C₁ à C₄, (dont chacun est éventuellement substitué par du fluor et/ou du chlore), par un radical diméthylaminosulfonyle ou diéthylaminosulfonyle, par un radical (alkoxy en C₁ à C₄)-carbonyle (qui est éventuellement substitué par du fluor, du chlore, du brome, un radical méthoxy ou éthoxy), par un radical phényle, phénylméthyle, phénoxy, phénylthio, phénylsulfinyle ou phényl-sulfonyle (dont chacun est éventuéllement substitué par du fluor, du chlore, du brome, un radical cyano, méthyle, méthoxy, trifluorométhyle, difluorométhoxy et/ou trifluorométhoxy),
A représente un groupe alcanediyle ou un groupe alcène-diyle ayant chacun 4 atomes de carbone,
E représente l'azote et
G représente un atome de carbone qui est lié à l'oxygène par une double liaison exo.

2. 4-cyanophényliminohétérocycles de formule générale (I) suivant la revendication 1, **caractérisés en ce que**
R¹ représente l'hydrogène, le fluor ou le chlore,
R² est un groupe -N(R⁴)-SO₂R⁵,
R⁴ représente l'hydrogène, un groupe méthyle, éthyle, n-propyle, isopropyle, n-butyle, isobutyle ou sec.butyle,
R⁵ représente un groupe méthyle, éthyle, n-propyle, iso-propyle, n-butyle, isobutyle, sec.-butyle ou tertio-butyle, dont chacun est éventuellement substitué par du fluor, du chlore, du brome, un radical cyano, carboxy, méthoxy, éthoxy, méthoxyéthoxy, éthoxyéthoxy, méthylthio, éthylthio, acétyle, propionyle, méthoxy-carbonyle ou éthoxycarbonyle,
R⁵ représente en outre un groupe allyle, crotonyle, 1-méthylallyle, propargyle ou 1-méthylpropargyle, chacun étant éventuellement substitué par du fluor ou du chlore,
R⁵ représente en outre un groupe cyclopropyle, cyclobutyle, cyclopentyle, cyclohexyle, cyclopropylméthyle, cyclobutylméthyle, cyclopentylméthyle ou cyclohexylméthyle, chacun étant éventuellement substitué par du fluor, du chlore, un radical méthyle, éthyle, n-propyle ou isopropyle,
R⁵ représente en outre un groupe phényle, naphtyle, benzyle ou phényléthyle dont chacun est éventuellement substitué par du fluor, du chlore, du brome, un radical cyano, méthyle, éthyle, n-propyle, isopropyle, n-butyle, isobutyle, sec.-butyle, tertio-butyle, méthoxy, éthoxy, méthylthio, éthylthio, méthyl-sulfinyle, éthylsulfinyle, méthylsulfonyle, éthyl-sulfonyle, trifluorométhyle, difluorométhoxy, trifluorométhoxy, trifluorométhylthio, trifluoro-méthylsulfinyle, trifluorométhylsulfonyle, diméthyl-aminosulfonyle, méthoxycarbonyle, éthoxycarbonyle, phényle, phénoxy ou phénylsulfonyle,
A représente un groupe butane-1,4-diyle (tétraméthylène), 1-butène-1,4-diyle ou 2-butène-1,4-diyle,
E représente l'azote et
G représente un atome de carbone qui est lié à l'oxygène par une double liaison exo.

3. 4-cyanophényliminohétérocycles suivant la revendication 1, **caractérisés par** la formule générale (IB) 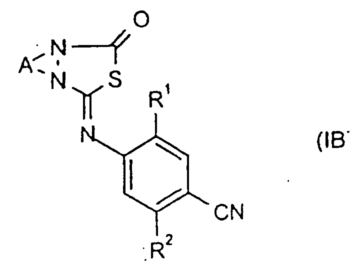 dans laquelle
R¹, R² et A ont les définitions indiquées dans la revendication 1.

4. Procédé de production de 4-cyanophényliminohétérocycles de formule générale (I) suivent la revendication 1, **caractérisé en ce que** :
a) on fait réagir des composés thiocarbonylaminés substitués de formule générale (III) 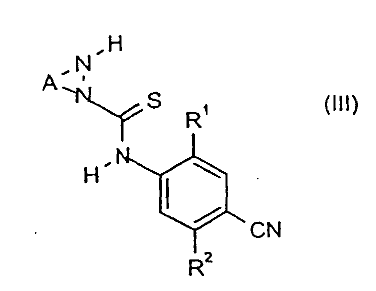 dans laquelle
A, R¹ et R² ont les définitions indiquées dans la revendication 1,
avec le phosgène, éventuellement en présence d'un diluant,
ou bien
b) on fait réagir des cyanaryliminohétérocycles de formule générale (Ia) 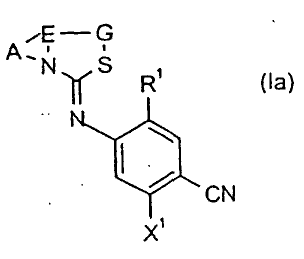
dans laquelle
R¹, A, E et G ont les définitions indiquées dans la reendication 1 et
X¹ est un halogène,
avec des composés nucléophiles de formule générale (V)
H-N(R⁴)-SO₂R⁵ (V)
dans laquelle
R⁴ et R⁵ ont les définitions indiquées dans la revendication 1,
- ou avec des sels de métaux alcalins de ces composés -
le cas échéant en présence d'un accepteur d'acide et en présence éventuelle d'un diluant.

5. Compositions herbicides, **caractérisées par** une teneur en au moins un 4-cyanophényliminohétérocycles de formule (I) suivant l'une des revendications 1 à 3.

6. Procédé de lutte contre des plantes indésirables, **caractérisé en ce qu'**on fait agir sur les plantes et/ou sur leur milieu des 4-cyanophényliminohétérocycles de formule générale (I) suivant les revendications 1 à 3.

7. Utilisation de 4-cyanophényliminohétérocycles de formule générale (I) suivant l'une des revendications 1 à 3 pour la lutte contre des plantes indésirables.

8. Procédé de préparation de compositions herbicides, **caractérisé en ce qu'**on mélange des 4-cyanophényliminohétérocycles de formule générale (I) suivant l'une des revendications 1 à 3 avec des diluants et/ou des substances tensio-actives.

9. Composés thiocarbonylaminés substitués de formule générale (III) 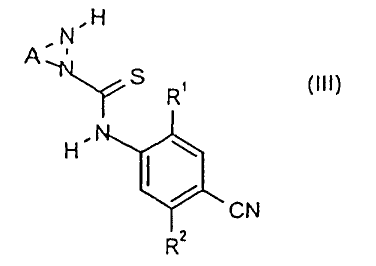 dans laquelle R¹, R² et A ont les définitions indiquées dans la revendication 1.
